# EUROPEAN PATENT SPECIFICATION

(11) **EP 3 272 737 B1**
(45) Date of publication and mention of the grant of the patent: **24.07.2019**
(21) Application number: 15885453.9
(22) Date of filing: 18.03.2015
(51) Int. Cl.: C07D 221/14, C08G 59/38, G03F 7/004

(54) **SULFONIC ACID DERIVATIVE COMPOUND, PHOTOACID GENERATOR, RESIST COMPOSITION, CATIONIC POLYMERIZATION INITIATOR, AND CATIONICALLY POLYMERIZABLE COMPOSITION**
SULFONSÄUREDERIVATVERBINDUNG, PHOTOSÄUREGENERATOR, RESISTZUSAMMENSETZUNG, KATIONISCHER POLYMERISATIONSINITIATOR UND KATIONISCH POLYMERISIERBARE ZUSAMMENSETZUNG
COMPOSÉ DE TYPE DÉRIVÉ D'ACIDE SULFONIQUE, GÉNÉRATEUR DE PHOTOACIDE, COMPOSITION DE RÉSERVE, INITIATEUR DE POLYMÉRISATION CATIONIQUE, ET COMPOSITION POLYMÉRISABLE PAR VOIE CATIONIQUE

(43) Date of publication of application: 24.01.2018
(73) Proprietor: Adeka Corporation, Tokyo 116-0012 (JP)
(72) Inventor: YANAGISAWA, Satoshi, Tokyo 116-0012 (JP); KIMURA, Masaki, Tokyo 116-0012 (JP); TODA, Hitomi, Tokyo 116-0012 (JP); SHIGENO, Koichi, Tokyo 116-0012 (JP)
(74) Representative: Müller-Boré & Partner Patentanwälte PartG mbB
(86) International application number: PCT/JP2015/058120
(87) International publication number: WO 2016/147357

(56) References cited:
- EP-A1- 2 927 216
- EP-A1- 3 018 182
- WO-A1-2011/087011
- WO-A1-2014/084269
- WO-A1-2015/001804
- JP-A- 2004 217 748
- US-A1- 2013 134 426

## Description

### TECHNICAL FIELD

The present invention relates to a sulfonic acid derivative compound, a photoacid generator, a resist composition, a cationic polymerization initiator, and a cationically polymerizable composition. More particularly, the present invention relates to: a sulfonic acid derivative compound which has high absorbance for light having a wavelength of 365 nm and exhibits high solubility in organic solvents and good acid generation rate; a photoacid generator; a resist composition; a cationic polymerization initiator; and a cationically polymerizable composition.

### BACKGROUND ART

Sulfonyloxyimides having a naphthalimino group which is a radioactive functional group are substances that generate an acid when irradiated with an energy beam such as light, and they are used as, for example, photoacid generators in photolithography resist compositions used for the formation of an electronic circuit such as a semiconductor, and as cationic polymerization initiators contained in photopolymerizable compositions such as resin compositions for stereolithography, paints, coatings, adhesives and inks.

For example, Patent Document 1 discloses a curable composition which comprises an acid-curable resin and a latent curing catalyst having a prescribed structure. It is disclosed that, in the latent curing catalyst having a prescribed structure, R¹ to R⁴ which are substituents of the naphthalene skeleton are each a hydrogen atom, an alkyl group having 1 to 8 carbon atoms, an alkoxy group having 1 to 4 carbon atoms, an alkylthio group having 1 to 12 carbon atoms, a nitro group, or a halogen atom.

In addition, Patent Document 2 discloses a photoresist comprising a sulfonyloxyimide having a prescribed structure as a sulfonic acid precursor, which photoresist is used in an ultraviolet radiation, electron beam or X-ray exposure apparatus. In Patent Document 2, naphthalimide, 3-nitronaphthalimide, 4-nitronaphthalimide, 4-chloronaphthalimide and 4-bromonaphthalimide are disclosed as naphthalimides.

Further, Patent Document 3 discloses an active ray-curable ink composition which comprises a sulfonic acid generator having a prescribed structure. As R₁ and R₂ which are substituents of the naphthalene skeleton in the sulfonic acid generator having a prescribed structure, alkyl groups, alkoxy groups, a carbonyl group, a phenylthio group, halogen atoms, a cyano group, a nitro group and a hydroxy group are disclosed.

Moreover, Patent Document 4 discloses an undercoat composition for a photoresist, a fluoride sulfonyloxyimide having a naphthalimino group as a photoactive compound, and alkyl groups having 1 to 8 carbon atoms and alkoxy groups having 1 to 8 carbon atoms are disclosed therein as substituents of the naphthalene skeleton.

### RELATED ART DOCUMENTS

### PATENT DOCUMENTS

Patent Document 1: Japanese Unexamined Patent Application Publication No. S57-151651
Patent Document 2: Japanese Unexamined Patent Application Publication (Translation of PCT Application) No. H8-501890
Patent Document 3: Japanese Unexamined Patent Application Publication No. 2004-217748
Patent Document 4: Japanese Unexamined Patent Application Publication (Translation of PCT Application) No. 2009-516207

### SUMMARY OF THE INVENTION

### PROBLEMS TO BE SOLVED BY THE INVENTION

As a light source for a photoacid generator used in photoresists or a cationic polymerization initiator used in resin compositions for stereolithography, adhesives, inks and the like, a far-ultraviolet radiation (e.g., EUV (Extreme Ultra-Violet), X-ray, F₂, ArF, KrF, i-line, h-line or g-line), an electron beam or a radioactive ray is often used. For the use of these light sources, materials showing high absorption at a wavelength of 365 nm are advantageous. Further, from the standpoint of conforming to high-precision patterning as well as shortening of the process, it is desired, in a photoresist or cationic polymerization system, to incorporate an adequate amount of an acid generator or to use an acid generator having good acid generation rate. Accordingly, there is a demand for an acid generator which is highly soluble in organic solvents and/or has sufficient acid generation rate.

However, as for the compound disclosed in Patent Document 1, only the case where R¹ to R⁴ are hydrogen atoms is described, and Patent Document 1 does not offer any disclosure or suggestion with regard to the differences in properties and performance that are attributed to the differences in types of these substituents, number of substitutions, positions of substitutions and the like. Moreover, as for the compound disclosed in Patent Document 4, Patent Document 4 does not offer any disclosure or suggestion with regard to the differences in properties and performance that are attributed to the differences in types of substitutions, positions of substitutions and the like. In this manner, the relationships between the type or position of a substituent and the acid generation rate of a sulfonic acid derivative compound were not sufficiently examined in Patent Documents 1 to 4.

In view of the above, an object of the present invention is to provide: a sulfonic acid derivative compound which has high absorbance for light having a wavelength of 365 nm and exhibits high solubility in organic solvents and good acid generation rate; a photoacid generator; a resist composition; a cationic polymerization initiator; and a cationically polymerizable composition.

### MEANS FOR SOLVING THE PROBLEMS

The present inventors intensively studied to solve the above-described problems and consequently discovered that a sulfonic acid derivative compound having a prescribed structure has high absorbance for light having a wavelength of 365 nm and exhibits high solubility in organic solvents and good acid generation rate, thereby completing the present invention.

That is, the sulfonic acid derivative compound of the present invention is characterized in that it is represented by the following Formula (I): 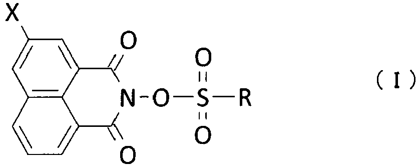 (wherein, X represents a linear or branched alkyl group having 1 to 14 carbon atoms; and R represents an aliphatic hydrocarbon group having 1 to 18 carbon atoms, an aryl group having 6 to 20 carbon atoms, an arylalkyl group having 7 to 20 carbon atoms, an acyl group-substituted aryl group having 7 to 20 carbon atoms, an alicyclic hydrocarbon group having 3 to 12 carbon atoms, a 10-camphoryl group, or a group represented by the following Formula (II), which aliphatic hydrocarbon group, aryl groups, arylalkyl group and alicyclic hydrocarbon group are optionally substituted with a group selected from the group consisting of a halogen atom, a halogenated alkyl group having 1 to 4 carbon atoms, an alkoxy group having 1 to 18 carbon atoms and an alkylthio group having 1 to 18 carbon atoms: 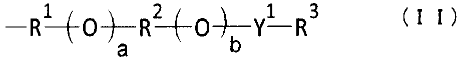 (wherein, Y¹ represents a single bond or an alkanediyl group having 1 to 4 carbon atoms; R¹ and R² each independently represent an alkanediyl group having 2 to 6 carbon atoms, a halogenated alkanediyl group having 2 to 6 carbon atoms, an arylene group having 6 to 20 carbon atoms, or a halogenated arylene group having 6 to 20 carbon atoms; R³ represents a linear or branched alkyl group having 1 to 18 carbon atoms, a linear or branched halogenated alkyl group having 1 to 18 carbon atoms, an alicyclic hydrocarbon group having 3 to 12 carbon atoms, an aryl group having 6 to 20 carbon atoms, a halogenated aryl group having 6 to 20 carbon atoms, an arylalkyl group having 7 to 20 carbon atoms, or a halogenated arylalkyl group having 7 to 20 carbon atoms; a and b each represent 0 or 1; and either a or b is 1)).

In the sulfonic acid derivative compound of the present invention, it is preferred that the X be an alkyl group having 4 carbon atoms and that the R be a perfluoroalkyl group having 1 to 8 carbon atoms.

The photoacid generator of the present invention is characterized by comprising the sulfonic acid derivative compound of the present invention.

The resist composition of the present invention is characterized by comprising the photoacid generator of the present invention.

The cationic polymerization initiator of the present invention is characterized by comprising the sulfonic acid derivative compound of the present invention.

The cationically polymerizable composition of the present invention is characterized by comprising the cationic polymerization initiator of the present invention.

### EFFECTS OF THE INVENTION

According to the present invention, a sulfonic acid derivative compound which has high absorbance for light having a wavelength of 365 nm and exhibits high solubility in organic solvents and good acid generation rate, a photoacid generator, a resist composition, a cationic polymerization initiator, and a cationically polymerizable composition can be provided.

### MODE FOR CARRYING OUT THE INVENTION

The present invention will now be described in detail based on embodiments thereof.

The sulfonic acid derivative compound of the present invention is represented by the following Formula (I): 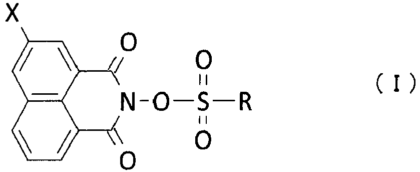 (wherein, X represents a linear or branched alkyl group having 1 to 14 carbon atoms; and R represents an aliphatic hydrocarbon group having 1 to 18 carbon atoms, an aryl group having 6 to 20 carbon atoms, an arylalkyl group having 7 to 20 carbon atoms, an acyl group-substituted aryl group having 7 to 20 carbon atoms, an alicyclic hydrocarbon group having 3 to 12 carbon atoms, a 10-camphoryl group, or a group represented by the following Formula (II), which aliphatic hydrocarbon group, aryl groups, arylalkyl group and alicyclic hydrocarbon group are optionally substituted with a halogen atom or a group selected from the group consisting of a halogenated alkyl group having 1 to 4 carbon atoms, an alkoxy group having 1 to 18 carbon atoms and an alkylthio group having 1 to 18 carbon atoms: 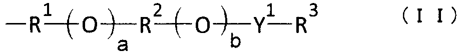 (wherein, Y¹ represents a single bond or an alkanediyl group having 1 to 4 carbon atoms; R¹ and R² each independently represent an alkanediyl group having 2 to 6 carbon atoms, a halogenated alkanediyl group having 2 to 6 carbon atoms, an arylene group having 6 to 20 carbon atoms, or a halogenated arylene group having 6 to 20 carbon atoms; R³ represents a linear or branched alkyl group having 1 to 18 carbon atoms, a linear or branched halogenated alkyl group having 1 to 18 carbon atoms, an alicyclic hydrocarbon group having 3 to 12 carbon atoms, an aryl group having 6 to 20 carbon atoms, a halogenated aryl group having 6 to 20 carbon atoms, an arylalkyl group having 7 to 20 carbon atoms, or a halogenated arylalkyl group having 7 to 20 carbon atoms; a and b each represent 0 or 1; and either a or b is 1)).

The sulfonic acid derivative compound of the present invention is structurally characterized by having a linear or branched alkyl group having 1 to 14 carbon atoms at a specific position (3-position of the naphthalene structure) of a naphthalimide skeleton of a photosensitive group. This structure increases the absorption (molar extinction coefficient, ε) at a wavelength of 365 nm, imparts the compound with good acid generation rate and improves the solubility in organic solvents. Such effects cannot be obtained when X is a hydrogen atom. For example, when X has more than 14 carbon atoms, although the solubility is increased, the molecular weight of the compound is increased and an acid generation rate adequate for the amount of use cannot be maintained.

In the Formula (I), examples of the linear or branched alkyl group having 1 to 14 carbon atoms which is represented by X include methyl, ethyl, propyl, isopropyl, 1-butyl, 2-butyl, isobutyl, *tert*-butyl, 1-pentyl, isopentyl, *tert*-pentyl, neopentyl, 1-hexyl, 2-hexyl, 3-hexyl, heptyl, 2-heptyl, 3-heptyl, isoheptyl, *tert*-heptyl, 1-octyl, isooctyl, *tert*-octyl, 2-ethylhexyl, 1-nonyl, isononyl, 1-decyl, and 1-dodecyl. Thereamong, alkyl group having 3 to 8 carbon atoms are preferred and alkyl groups having 4 carbon atoms are more preferred, because these alkyl groups have both good solubility and good acid generation rate. A 1-butyl group is still more preferred because the material is inexpensive and has good yield and low production cost. It is yet still more preferred that X be an unsubstituted alkyl group.

In the Formula (I), examples of the aliphatic hydrocarbon group having 1 to 18 carbon atoms which is represented by R include an alkenyl group, an alkyl group, a group in which a methylene group in an alkyl group is substituted with an alicyclic hydrocarbon group, a group in which a proton of a methylene group in an alkyl group is substituted with an alicyclic hydrocarbon group, and a group in which an alicyclic hydrocarbon exists at a terminal of an alkyl group. Examples of the alkenyl group include allyl and 2-methyl-2-propenyl, and examples of the alkyl group include methyl, ethyl, propyl, isopropyl, butyl, *sec*-butyl, *tert*-butyl, isobutyl, pentyl, isopentyl, *tert*-pentyl, hexyl, 2-hexyl, 3-hexyl, heptyl, 2-heptyl, 3-heptyl, isoheptyl, *tert*-heptyl, octyl, isooctyl, *tert*-octyl, 2-ethylhexyl, nonyl, isononyl, decyl, dodecyl, tridecyl, tetradecyl, pentadecyl, hexadecyl, heptadecyl, and octadecyl. Examples of the alicyclic hydrocarbon group include cyclopropane, cyclobutane, cyclopentane, cyclohexane, cycloheptane, cyclooctane, cyclodecane, bicyclo[2.1.1]hexane, bicyclo[2.2.1]heptane, bicyclo[3.2.1]octane, bicyclo[2.2.2]octane, and adamantane.

In the Formula (I), examples of the aryl group having 6 to 20 carbon atoms which is represented by R include phenyl, naphthyl, 2-methylphenyl, 3-methylphenyl, 4-methylphenyl, 4-vinylphenyl, 3-isopropylphenyl, 4-isopropylphenyl, 4-butylphenyl, 4-isobutylphenyl, 4-*tert*-butylphenyl, 4-hexylphenyl, 4-cyclohexylphenyl, 4-octylphenyl, 4-(2-ethylhexyl)phenyl, 2,3-dimethylphenyl, 2,4-dimethylphenyl, 2,5-dimethylphenyl, 2,6-dimethylphenyl, 3,4-dimethylphenyl, 3,5-dimethylphenyl, 2,4-di-*tert*-butylphenyl, 2,5-di-*tert*-butylphenyl, 2,6-di-*tert*-butylphenyl, 2,4-di-*tert*-pentylphenyl, 2,5-di-*tert*-pentylphenyl, 2,5-di-*tert*-octylphenyl, cyclohexylphenyl, biphenyl, 2,4,5-trimethylphenyl, 2,4,6-trimethylphenyl, and 2,4,6-triisopropylphenyl.

In the Formula (I), examples of the arylalkyl group having 7 to 20 carbon atoms which is represented by R include benzyl, phenethyl, 2-phenylpropan-2-yl, diphenylmethyl, and triphenylmethyl.

In the Formula (I), the number of carbon atoms of the acyl group-substituted aryl group having 7 to 20 carbon atoms which is represented by R includes the carbon atoms of the acyl group. Examples of such an aryl group include acetylphenyl, acetylnaphthyl, benzoylphenyl, 1-anthraquinolyl, and 2-anthraquinolyl.

In the Formula (I), examples of the alicyclic hydrocarbon group having 3 to 12 carbon atoms which is represented by R include cyclopropane, cyclobutane, cyclopentane, cyclohexane, cycloheptane, cyclooctane, cyclodecane, bicyclo[2.1.1]hexane, bicyclo[2.2.1]heptane, bicyclo[3.2.1]octane, bicyclo[2.2.2]octane, and adamantane.

In the Formula (I), the aliphatic hydrocarbon group having 1 to 18 carbon atoms, the aryl group having 6 to 20 carbon atoms, the arylalkyl group having 7 to 20 carbon atoms, the acyl group-substituted aryl group having 7 to 20 carbon atoms and the alicyclic hydrocarbon group having 3 to 12 carbon atoms, which are represented by R, are optionally substituted with a halogen atom or a group selected from the group consisting of a halogenated alkyl group having 1 to 4 carbon atoms, an alkoxy group having 1 to 18 carbon atoms and an alkylthio group having 1 to 18 carbon atoms. Examples of the halogen atom include chlorine, bromine, iodine, and fluorine.

In the Formula (I), examples of the aliphatic hydrocarbon group having 1 to 18 carbon atoms which is represented by R and substituted with a halogen atom include halogenated alkyl groups such as trifluoromethyl, pentafluoroethyl, 2-chloroethyl, 2-bromoethyl, heptafluoropropyl, 3-bromopropyl, nonafluorobutyl, tridecafluorohexyl, heptadecafluorooctyl, 2,2,2-trifluoroethyl, 1,1-difluoroethyl, 1,1-difluoropropyl, 1,1,2,2-tetrafluoropropyl, 3,3,3-trifluoropropyl, 2,2,3,3,3-pentafluoropropyl, norbornyl-1,1-difluoroethyl, norbornyltetrafluoroethyl, adamantane-1,1 ,2,2-tetrafluoropropyl, and bicyclo[2.2.1]heptane-tetrafluoromethyl.

In the Formula (I), examples of the aryl group having 6 to 20 carbon atoms which is represented by R and substituted with a halogen atom include pentafluorophenyl, chlorophenyl, dichlorophenyl, trichlorophenyl, 2,4-bis(trifluoromethyl)phenyl, and bromoethylphenyl.

In the Formula (I), examples of the arylalkyl group having 7 to 20 carbon atoms which is represented by R and substituted with a halogen atom include pentafluorophenylmethyl, phenyldifluoromethyl, 2-phenyl-tetrafluoroethyl, and 2-(pentafluorophenyl)ethyl. Examples of the arylalkyl group having 7 to 20 carbon atoms which is substituted with an alkylthio group having 1 to 18 carbon atoms include *p*-methylthiobenzyl. Examples of the arylalkyl group which is substituted with both a halogen atom and an alkylthio group having 1 to 18 carbon atoms include 2,3,5,6-tetrafluoro-4-methylthiophenylethyl.

Examples of the halogenated alkyl group having 1 to 4 carbon atoms include chloromethyl, bromomethyl, iodomethyl, fluoromethyl, dichloromethyl, dibromomethyl, difluoromethyl, trichloromethyl, tribromomethyl, trifluoromethyl, 2-chloroethyl, 2-bromoethyl, 2-fluoroethyl, 1,2-dichloroethyl, 2,2-difluoroethyl, 1-chloro-2-fluoroethyl, 3-chloro-*n*-propyl, 3-bromo-*n*-propyl, 3-fluoro-*n*-propyl, and 4-chloro-*n*-butyl.

Examples of the alkoxy group having 1 to 18 carbon atoms include methoxy, ethoxy, propoxyl, butoxy, *tert*-butoxy, pentyloxy, hexyloxy, heptyloxy, octyloxy, nonyloxy, decyloxy, undecyloxy, dodecyloxy, tridecyloxy, tetradecyloxy, pentadecyloxy, hexadecyloxy, heptadecyloxy, and octadecyloxy.

Examples of the alkylthio group having 1 to 18 carbon atoms include methylthio, ethylthio, propylthio, isopropylthio, butylthio, *sec*-butylthio, *tert*-butylthio, isobutylthio, pentylthio, isopentylthio, *tert*-pentylthio, hexylthio, heptylthio, isoheptylthio, *tert*-heptylthio, octylthio, isooctylthio, *tert*-octylthio, 2-ethylhexylthio, nonylthio, decylthio, undecylthio, dodecylthio, tridecylthio, tetradecylthio, pentadecylthio, hexadecylthio, heptadecylthio, and octadecylthio.

Examples of the aliphatic hydrocarbon having 1 to 18 carbon atoms which is substituted with an alkylthio group having 1 to 18 carbon atoms include 2-methylthioethyl, 4-methylthiobutyl and 4-butylthioethyl, and examples of the aliphatic hydrocarbon having 1 to 18 carbon atoms which is substituted with both a halogen atom and an alkylthio group having 1 to 18 carbon atoms include 1,1,2,2-tetrafluoro-3-methylthiopropyl.

Examples of the aryl group having 6 to 20 carbon atoms which is substituted with an alkylthio group having 1 to 18 carbon atoms include 4-methylthiophenyl, 4-butylthiophenyl, 4-octylthiophenyl, and 4-dodecylthiophenyl. Examples of the aryl group having 6 to 20 carbon atoms which is substituted with both a halogen atom and an alkylthio group having 1 to 18 carbon atoms include 1,2,5,6-tetrafluoro-4-methylthiophenyl, 1,2,5,6-tetrafluoro-4-butylthiophenyl, and 1,2,5,6-tetrafluoro-4-dodecylthiophenyl.

The Formula (II) is an ether group. In the Formula (II), Y¹ represents a single bond or an alkanediyl group having 1 to 4 carbon atoms; R¹ and R² each independently represent an alkanediyl group having 2 to 6 carbon atoms, a halogenated alkanediyl group having 2 to 6 carbon atoms, an arylene group having 6 to 20 carbon atoms, or a halogenated arylene group having 6 to 20 carbon atoms; R³ represents a linear or branched alkyl group having 1 to 18 carbon atoms, a linear or branched halogenated alkyl group having 1 to 18 carbon atoms, an alicyclic hydrocarbon group having 3 to 12 carbon atoms, an aryl group having 6 to 20 carbon atoms, a halogenated aryl group having 6 to 20 carbon atoms, an arylalkyl group having 7 to 20 carbon atoms, or a halogenated arylalkyl group having 7 to 20 carbon atoms; a and b each represent 0 or 1; and either a or b is 1.

In the Formula (II), examples of the alkanediyl group having 1 to 4 carbon atoms which is represented by Y¹ include methylene, ethylene, propane-1,3-diyl, propane- 1,2-diyl, butylene, butane-1,3-diyl, butane-2,3-diyl, and butane-1,2-diyl.

In the Formula (II), examples of the alkanediyl group having 2 to 6 carbon atoms which is represented by R¹ and R² include ethylene, propane-1,3-diyl, propane-1,2-diyl, butylene, butane-1,3-diyl, butane-2,3-diyl, butane-1,2-diyl, pentane-1,5-diyl, pentane-1,3-diyl, pentane-1,4-diyl, pentane-2,3-diyl, hexane-1,6-diyl, hexane-1,2-diyl, hexane-1,3-diyl, hexane-1,4-diyl, hexane-2,5-diyl, hexane-2,4-diyl, and hexane-3,4-diyl.

In the Formula (II), the halogenated alkanediyl group having 2 to 6 carbon atoms, which is represented by R¹ and R², is any one of the above-described alkanediyl groups having 1 to 6 carbon atoms in which at least one hydrogen atom is substituted with a halogen atom. Examples of the halogen atom include chlorine, bromine, iodine, and fluorine. Examples of the halogenated alkanediyl group having 2 to 6 carbon atoms include tetrafluoroethylene, 1,1-difluoroethylene, 1-fluoroethylene, 1,2-difluoroethylene, hexafluoropropane-1,3-diyl, 1,1,2,2-tetrafluoropropane-1,3-diyl, and 1,1,2,2-tetrafluoropentane-1,5-diyl.

In the Formula (II), examples of the arylene group having 6 to 20 carbon atoms which is represented by R¹ and R² include 1,2-phenylene, 1,3-phenylene, 1,4-phenylene, 2,5-dimethyl-1,4-phenylene, 4,4'-biphenylene, diphenylmethane-4,4'-diyl, 2,2-diphenylpropane-4,4'-diyl, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, and naphthalene-2,7-diyl.

In the Formula (II), the halogenated arylene group having 6 to 20 carbon atoms, which is represented by R¹ and R², is any one of the above-described arylene groups having 6 to 20 carbon atoms in which at least one hydrogen atom is substituted with a halogen atom. Examples of the halogen atom include chlorine, bromine, iodine, and fluorine. Examples of the halogenated arylene group having 6 to 20 carbon atoms include tetrafluorophenylene.

In the Formula (II), examples of the linear or branched alkyl group having 1 to 18 carbon atoms which is represented by the R³ include methyl, ethyl, propyl, isopropyl, butyl, *sec-*butyl, *tert*-butyl, isobutyl, pentyl, isopentyl, *tert*-pentyl, hexyl, 2-hexyl, 3-hexyl, heptyl, 2-heptyl, 3-heptyl, isoheptyl, *tert*-heptyl, octyl, isooctyl, *tert*-octyl, 2-ethylhexyl, nonyl, isononyl, decyl, dodecyl, tridecyl, tetradecyl, pentadecyl, hexadecyl, heptadecyl, and octadecyl.

In the Formula (II), the linear or branched halogenated alkyl group having 1 to 18 carbon atoms, which is represented by R³, is any one of the above-described alkyl groups having 1 to 18 carbon atoms in which at least one hydrogen atom is substituted with a halogen atom. Examples of the halogen atom include chlorine, bromine, iodine, and fluorine. Examples of the linear or branched halogenated alkyl group having 1 to 18 carbon atoms include halogenated alkyl groups such as trifluoromethyl, pentafluoroethyl, heptafluoropropyl, nonafluorobutyl, tridecafluorohexyl, heptadecafluorooctyl, 2,2,2-trifluoroethyl, 1,1-difluoroethyl, 1,1-difluoropropyl, 1,1,2,2-tetrafluoropropyl, 3,3,3-trifluoropropyl, 2,2,3,3,3-pentafluoropropyl, and 1,1,2,2-tetrafluorotetradecyl.

In the Formula (II), examples of the alicyclic hydrocarbon group having 3 to 12 carbon atoms which is represented by R³ include, stating them in terms of the name of the cycloalkane constituting the respective alicyclic hydrocarbon groups: cyclopropane, cyclobutane, cyclopentane, cyclohexane, cycloheptane, cyclooctane, cyclodecane, bicyclo[2.1.1]hexane, bicyclo[2.2.1]heptane, bicyclo[3.2.1]octane, bicyclo[2.2.2]octane, and adamantane.

In the Formula (II), examples of the aryl group having 6 to 20 carbon atoms, halogenated aryl group having 6 to 20 carbon atoms, arylalkyl group having 7 to 20 carbon atoms and halogenated arylalkyl group having 7 to 20 carbon atoms, which are represented by the R³, include the same groups as those exemplified above for R in the Formula (I).

Specific examples of the sulfonic acid derivative compound of the present invention include the following Compound Nos. 1 to 43.

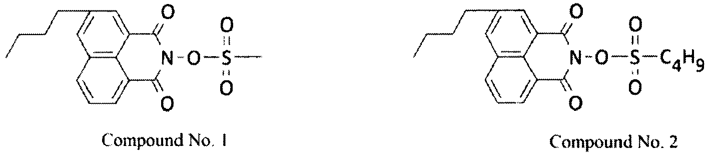

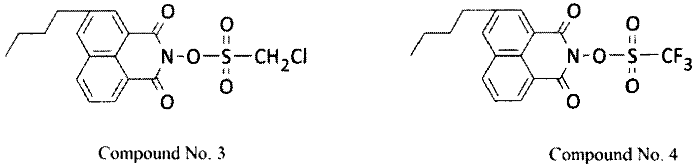

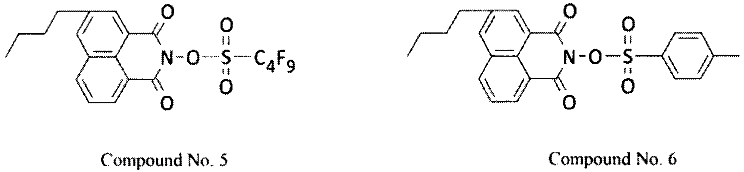

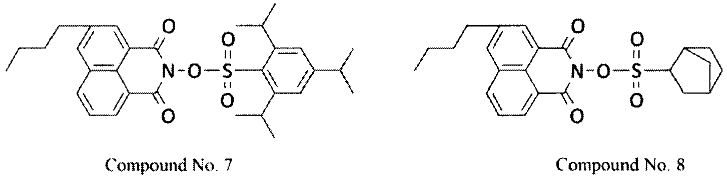

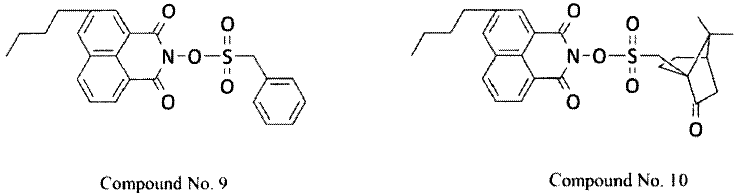

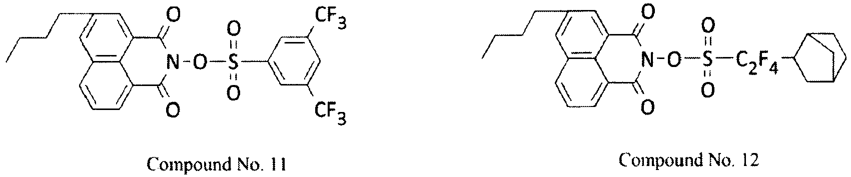

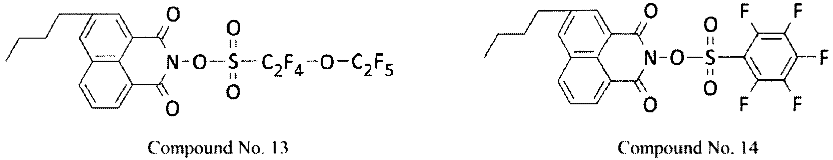

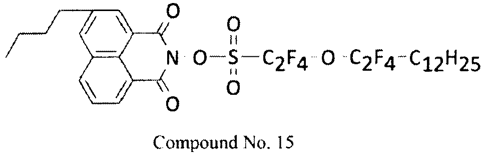

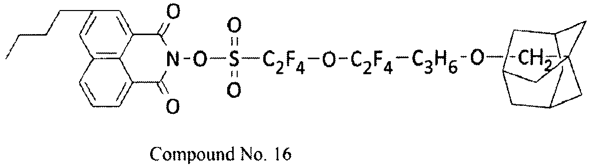

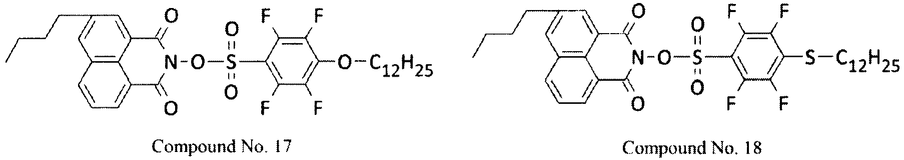

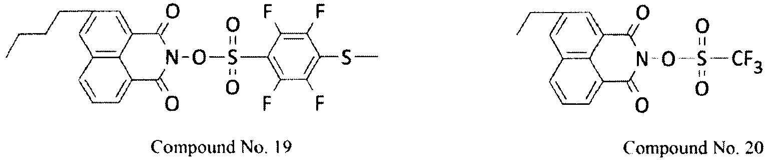

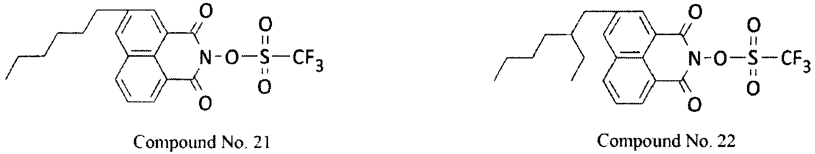

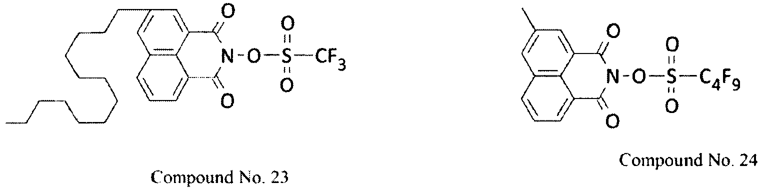

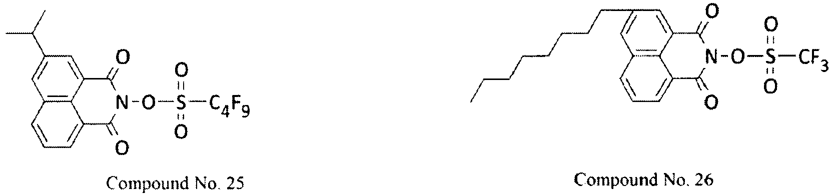

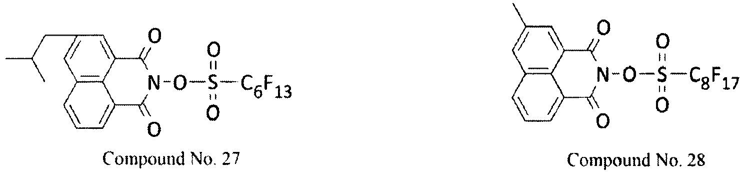

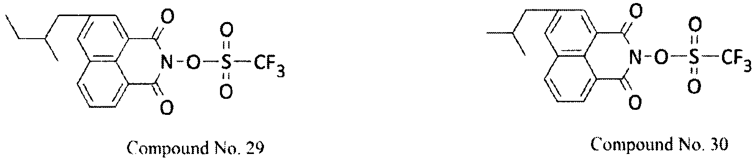

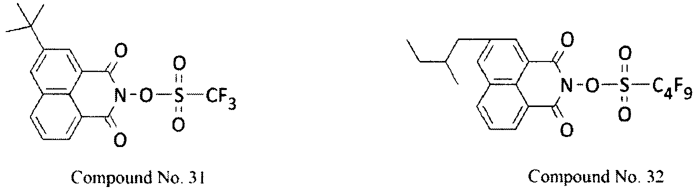

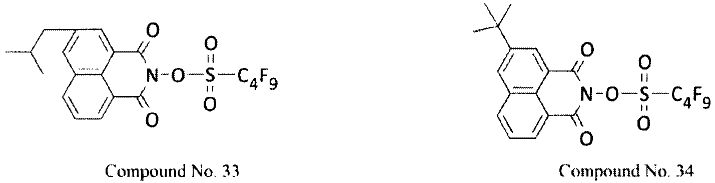

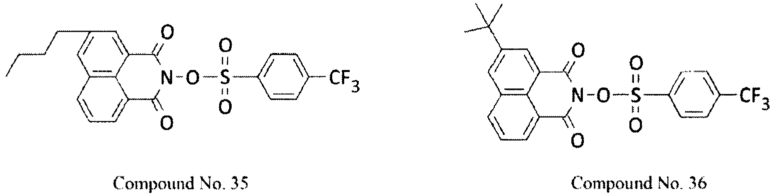

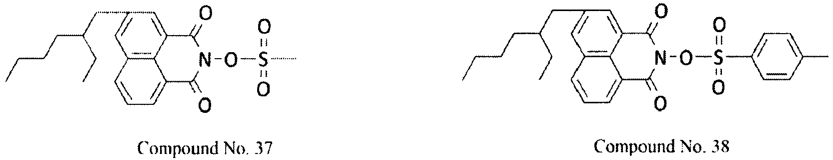

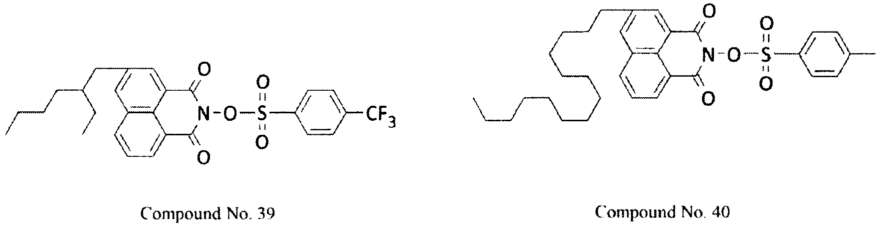

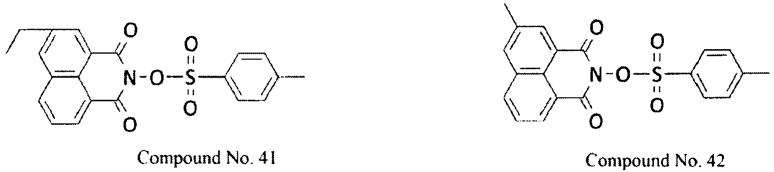

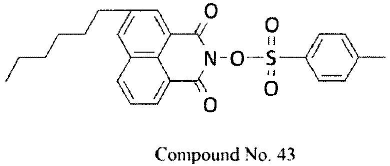

R in the Formula (I) may be selected such that it allows an organic sulfonic acid appropriate for the intended use to be released. For high-sensitive and high-precision patterning, perfluoroalkane sulfonic acid which has a high acid strength and provides high sensitivity is most useful. Accordingly, in the compound of the present invention, R is preferably a perfluoroalkyl group having 1 to 8 carbon atoms.

The method of producing the sulfonic acid derivative compound of the present invention is not particularly restricted, and a well-known chemical reaction can be applied to synthesize the sulfonic acid derivative compound of the present invention. For example, a method of synthesizing a sulfonic acid derivative compound using a bromide as a starting substance as described below can be employed. 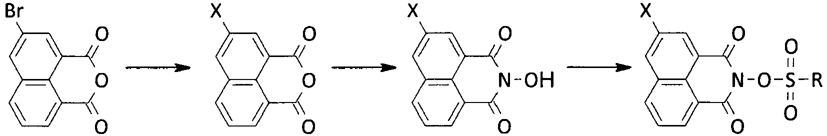 (wherein, X and R each represent the same group as in the Formula (I))

The sulfonic acid derivative compound of the present invention has a property of releasing a Lewis acid when irradiated with an active energy beam, such as a far ultraviolet ray (e.g., EUV (Extreme Ultra-Violet), X-ray, F₂, ArF, KrF, i-line, h-line or g-line), an electron beam, a radioactive ray or a high-frequency wave, and is capable of acting on an acid-reactive organic substance to induce decomposition or polymerization thereof. The sulfonic acid derivative compound of the present invention is thus useful as a photoacid generator of a positive or negative photoresist and as a cationic polymerization initiator used in a wide range of applications, including photoresists for the preparation of lithographic plates, letterpress printing plates, printed boards, ICs or LSIs, image formation such as relief image formation and image replication, and photocurable inks, paints, adhesives and the like.

Next, the photoacid generator of the present invention will be described.

The photoacid generator of the present invention comprises the sulfonic acid derivative compound of the present invention. The photoacid generator of the present invention can be used for, for example, cleaving a chemical bond of an ester group, an ether group or the like in acid-reactive organic substances or acrylic resins. When the photoacid generator of the present invention is used for an acid-reactive organic substance, the amount thereof is not particularly restricted; however, it is preferably 0.01 to 100 parts by mass, more preferably 0.05 to 20 parts by mass, with respect to 100 parts by mass of the acid-reactive organic substance. When the amount of the photoacid generator is less than 0.01 parts by mass, the sensitivity and the developability may be deteriorated, whereas when the amount is greater than 20 parts by mass, the transparency to radiation is reduced, which can makes it difficult to obtain a rectangular resist pattern. It is noted here, however, that the photoacid generator of the present invention may be used in an amount that is greater or less than the above-described range, depending on the factors such as the properties of the acid-reactive organic substance, the light irradiation intensity, the time required for reaction, the physical properties and the cost.

Next, the resist composition of the present invention will be described.

The resist composition of the present invention comprises the sulfonic acid derivative compound of the present invention as an indispensable photoacid generator along with a resin whose solubility in a developing solution changes with the action of an acid (hereinafter, such a resin is also referred to as "resist base resin"). The resist composition of the present invention is particularly useful as a chemically amplified resist. There are two types of chemically amplified resists: positive resists which are made soluble in a developing solution through a polarity change induced by a deprotection reaction of the side chain of a resist base resin, such as cleavage of a chemical bond of an ester group, an acetal group or the like, which is attributed to the action of an acid generated from a photoacid generator upon exposure; and negative resists which undergoes a chemical chain reaction such as polymerization or cross-linking and is made insoluble in a developing solution by a cross-linking reaction or polarity change and from which only unexposed parts are selectively removed upon development. In the present invention, such a resist base resin may be used individually, or two or more thereof may be used in combination.

The resist base resin used in the resist composition of the present invention is not particularly restricted; however, it preferably has a structure which has a small extinction coefficient for the wavelength of active energy beam and exhibits high etching resistance.

Examples of the resist base resin include polyhydroxystyrenes and derivatives thereof; polyacrylic acids and derivatives thereof; polymethacrylic acids and derivatives thereof; copolymers formed by two or more selected from hydroxystyrene, acrylic acid, methacrylic acid and derivatives thereof; copolymers formed by two or more selected from hydroxystyrene, styrene and derivatives thereof; copolymers formed by three or more selected from polyolefins and derivatives thereof, cycloolefins and derivatives thereof, maleic anhydride, and acrylic acid and derivatives thereof; copolymers formed by three or more selected from cycloolefins and derivatives thereof, maleimide, and acrylic acid and derivatives thereof; polynorbornenes; high-molecular-weight polymers of one or more selected from the group consisting of metathesis ring-opening polymers; and silicone resins.

Detailed specific examples of the resist base resin are disclosed in, for example, Claims 8 to 11 of Japanese Unexamined Patent Application Publication No. 2003-192665, Claim 3 of Japanese Unexamined Patent Application Publication No. 2004-323704, Japanese Unexamined Patent Application Publication No. H10-10733, Japanese Unexamined Patent Application Publication No. 2010-15079, and Japanese Unexamined Patent Application Publication No. 2010-15101.

The polystyrene-equivalent weight-average molecular weight (Mw) of the resist base resin, which is determined by gel permeation chromatography (GPC), is usually 1,000 to 500,000, preferably 2,000 to 200,000, more preferably 3,000 to 100,000. In this case, when the Mw of the resist base resin is less than 1,500, the resist base resin tends to show a reduced heat resistance as a resist, whereas when the Mw is higher than 300,000, the developability and coatability of the resist base resin as a resist tends to be deteriorated.

In a positive resist, a high-molecular-weight polymer obtained by introducing a protecting group, which is decomposed by the action of an acid, into the resist base resin is used. Examples of the protecting group include tertiary alkyl groups, trialkylsilyl groups, oxoalkyl groups, aryl group-substituted alkyl groups, heteroalicyclic groups such as a tetrahydropyran-2-yl group, tertiary alkylcarbonyl groups, tertiary alkylcarbonylalkyl groups, tertiary alkyloxycarbonyl groups, tertiary alkyloxycarbonylalkyl groups, alkoxyalkyl groups, and acetal groups such as a tetrahydropyranyl group, a tetrahydrofuranyl group and a thiofuranyl group.

In a negative resist, a resin obtained by a reaction between the resist base resin and a cross-linking agent is used. The cross-linking agent can be arbitrarily selected from those resins that are commonly used as cross-linking agents, and examples thereof include amino resins having a hydroxyl group or an alkoxyl group, such as melamine resins, urea resins, guanamine resins, glycoluril-formaldehyde resins, succinylamide-formaldehyde resins and ethylene urea-formaldehyde resins. As these cross-linking agents, melamine, urea, guanamine, glycoluril, succinylamide and ethylene urea that are each methylolated through reaction with formalin in boiling water, or the resultants thereof further alkoxylated through reaction with a lower alcohol, can be used.

As the cross-linking agent, a commercially available one can be used as well, and examples thereof include NIKALAC MX-750, NIKALAC MW-30, and NIKALAC MX-290 (manufactured by Sanwa Chemical Co., Ltd.).

When the sulfonic acid derivative compound of the present invention is used as a photoacid generator, other photoacid generator such as an iodonium salt compound or a sulfonium compound may also be used in combination. When other photoacid generator is used, it is preferably used in an amount of 10 to 200 parts by mass with respect to 100 parts by mass of the sulfonic acid derivative compound of the present invention.

In the resist composition of the present invention, in addition to a photoacid generator other than the sulfonic acid derivative compound of the present invention, a variety of resin additives, such as an unsaturated bond-containing monomer, a chain transfer agent, a surfactant, a thermoplastic organic polymer, a thermal polymerization inhibitor, a base quencher, an acid amplifier, an acid dispersant, a base generator, an inorganic filler, an organic filler, a coloring agent (e.g., pigment or dye), an antifoaming agent, a thickening agent, a flame retardant, a UV absorber, an antioxidant, a stabilizer, a sensitizer, a plasticizer, an adhesion promoter, an antistatic agent, a lubricant, a crystallizer, a dispersant, a leveling agent and a silane-coupling agent, can be incorporated. In the resist composition of the present invention, these additives are used in a total amount of preferably 50% by mass or less.

Prior to its use, the resist composition of the present invention is normally adjusted by being dissolved in a solvent to a total solid concentration of usually 5 to 50% by weight, preferably 10 to 25% by weight, and subsequently filtered through, for example, a filter having a pore size of about 0.2 µm. The resist composition of the present invention can be prepared by a method of, for example, mixing, dissolving or kneading a photoacid generator composed of the sulfonic acid derivative compound of the present invention, other photoacid generator(s), a resist base resin and other arbitrary component(s).

A light source used for the exposure of the above-described resist composition is selected as appropriate from those emitting g-line (436 nm), h-line (405 nm), i-line (365 nm), visible light, ultraviolet radiation, far-ultraviolet radiation, X-ray, charged particle beam or the like in accordance with the type of the photoacid generator(s) in use, and the sulfonic acid derivative compound of the present invention can be suitably applied to a resist which utilizes g-line (436 nm), h-line (405 nm), i-line (365 nm), visible light or the like.

The resist composition of the present invention is coated on a substrate made of silicon or the like by an appropriate coating method using a spinner, a coater or the like, subsequently exposed through a prescribed mask, post-baked for improvement of the apparent sensitivity of the resulting resist and then developed, whereby a more favorable resist pattern can be obtained.

Next, the cationic polymerization initiator of the present invention will be described.

The cationic polymerization initiator of the present invention comprises the sulfonic acid derivative compound of the present invention. The amount thereof to be used is not particularly restricted; however, it is preferably 0.01 to 100 parts by mass, more preferably 0.05 to 20 parts by mass, with respect to 100 parts by mass of a cationically polymerizable compound. In this case, when the cationic polymerization initiator is used in an amount of less than 0.01 parts by mass, the sensitivity may be reduced, whereas when the amount is greater than 20 parts by mass, the transparency to radiation may be deteriorated. It is noted here, however, that the cationic polymerization initiator of the present invention may be used in an amount that is greater or less than the above-described range, depending on the factors such as the properties of the cationically polymerizable compound, the light irradiation intensity, the time required for reaction, the physical properties and the cost.

Next, the cationically polymerizable composition of the present invention will be described.

The cationically polymerizable composition of the present invention is a composition which comprises the cationic polymerization initiator of the present invention and a cationically polymerizable compound. The term "cationically polymerizable compound" used herein means a compound whose polymerization or cross-linking reaction is induced by a cationic polymerization initiator activated by irradiation with light, and such a compound is used individually, or in combination of two or more thereof.

Examples of the cationically polymerizable compound include epoxy compounds, oxetane compounds, cyclic lactone compounds, cyclic acetal compounds, cyclic thioether compounds, spiro-orthoester compounds and vinyl compounds, and one or more of these compounds can be used. Thereamong, epoxy compounds and oxetane compounds are suitable because of their availability and ease of handling. As the epoxy compounds, for example, alicyclic epoxy compounds, aromatic epoxy compounds and aliphatic epoxy compounds are suitable.

Examples of the alicyclic epoxy compounds include polyglycidyl ethers of polyhydric alcohols having at least one alicyclic ring; and cyclohexene oxide or cyclopentene oxide-containing compounds obtained by epoxidation of a cyclohexene ring or cyclopentene ring-containing compound with an oxidizing agent. Examples of these compounds include hydrogenated bisphenol-A diglycidyl ether, 3,4-epoxycyclohexylmethyl-3,4-epoxycyclohexane carboxylate, 3,4-epoxy-1-methylcyclohexyl-3,4-epoxy-1-methylcyclohexane carboxylate, 6-methyl-3,4-epoxycyclohexylmethyl-6-methyl-3,4-epoxycyclohexane carboxylate, 3,4-epoxy-3-methylcyclohexylmethyl-3,4-epoxy-3-methylcyclohexane carboxylate, 3,4-epoxy-5-methylcyclohexylmethyl-3,4-epoxy-5-methylcyclohexane carboxylate, 2-(3,4-epoxycyclohexyl-5,5-spiro-3,4-epoxy)cyclohexane-metadioxane, bis(3,4-epoxycyclohexylmethyl)adipate, 3,4-epoxy-6-methylcyclohexyl carboxylate, methylene-bis(3,4-epoxycyclohexane), dicyclopentadiene diepoxide, ethylene-bis(3,4-epoxycyclohexanecarboxylate), dioctyl epoxyhexahydrophthalate, and di-2-ethylhexyl epoxyhexahydrophthalate.

Examples of commercially available products that can be suitably used as an alicyclic epoxy compound include UVR-6100, UVR-6105, UVR-6110, UVR-6128 and UVR-6200 (all of which are manufactured by Union Carbide Corporation); CELLOXIDE 2021, CELLOXIDE 2021P, CELLOXIDE 2081, CELLOXIDE 2083, CELLOXIDE 2085, CELLOXIDE 2000, CELLOXIDE 3000, CYCLOMER A200, CYCLOMER M100, CYCLOMER M101, EPOLEAD GT-301, EPOLEAD GT-302, EPOLEAD 401, EPOLEAD 403, ETHB and EPOLEAD HD300 (all of which are manufactured by Daicel Chemical Industries, Ltd.); and KRM-2110 and KRM-2199 (both of which are manufactured by ADEKA Corporation).

Among these alicyclic epoxy compounds, epoxy resins having a cyclohexene oxide structure are preferred because of their curability (curing rate).

Further, specific examples of the aromatic epoxy compounds include polyglycidyl ethers of polyhydric phenols having at least one aromatic ring or alkylene oxide adducts thereof, such as glycidyl ethers of bisphenol A, bisphenol F or an alkylene oxide adduct thereof; and epoxy novolac resins.

Specific examples of the aliphatic epoxy compounds include polyglycidyl ethers of aliphatic polyhydric alcohols or alkylene oxide adducts thereof; polyglycidyl esters of aliphatic long-chain polybasic acids; homopolymers synthesized by vinyl polymerization of glycidyl acrylate or glycidyl methacrylate; and copolymers synthesized by vinyl polymerization of glycidyl acrylate or glycidyl methacrylate and other vinyl monomer(s). Representative examples of these compounds include glycidyl ethers of polyhydric alcohols, such as 1,4-butanediol diglycidyl ether, 1,6-hexanediol diglycidyl ether, glycerol triglycidyl ether, trimethylolpropane triglycidyl ether, sorbitol tetraglycidyl ether, dipentaerythritol hexaglycidyl ether, polyethylene glycol diglycidyl ether and polypropylene glycol diglycidyl ether; polyglycidyl ethers of polyether polyols obtained by addition of one or more alkylene oxides to an aliphatic polyhydric alcohol, such as propylene glycol, trimethylolpropane and glycerin; and diglycidyl esters of aliphatic long-chain dibasic acids. Examples thereof also include monoglycidyl ethers of aliphatic higher alcohols; monoglycidyl ethers of phenol, cresol, butylphenol, or polyether alcohols obtained by addition of an alkylene oxide thereto; glycidyl esters of higher fatty acids; epoxidized soybean oil; octyl epoxystearate; butyl epoxystearate; and epoxidized polybutadienes.

Examples of commercially available products that can be suitably used as an aromatic compound or an aliphatic epoxy compound include EPIKOTE 801 (jER801) and EPIKOTE 828 (jER828) (both of which are manufactured by Mitsubishi Chemical Corporation); PY-306, 0163, and DY-022 (all of which are manufactured by BASF Japan, Ltd.); KRM-2720, EP-4100, EP-4000, EP-4080, EP-4900, ED-505 and ED-506 (all of which are manufactured by ADEKA Corporation); EPOLIGHT M-1230, EPOLIGHT EHDG-L, EPOLIGHT 40E, EPOLIGHT 100E, EPOLIGHT 200E, EPOLIGHT 400E, EPOLIGHT 70P, EPOLIGHT 200P, EPOLIGHT 400P, EPOLIGHT 1500NP, EPOLIGHT 1600, EPOLIGHT 80MF, EPOLIGHT 100MF, EPOLIGHT 4000, EPOLIGHT 3002 and EPOLIGHT FR-1500 (all of which are manufactured by Kyoeisha Chemical Co., Ltd.); and SUNTOHTO ST0000, YD-716, YH-300, PG-202, PG-207, YD-172 and YDPN638 (all of which are manufactured by Tohto Kasei Co., Ltd.).

Further, specific examples of the oxetane compounds include the following compounds: 3-ethyl-3-hydroxymethyloxetane, 3-(meth)allyloxymethyl-3-ethyloxetane, (3-ethyl-3-oxetanylmethoxy)methylbenzene, 4-fluoro-[1-(3-ethyl-3-oxetanylmethoxy)methyl]benzene, 4-methoxy-[1-(3-ethyl-3-oxetanylmethoxy)methyl]benzene, [1-(3-ethyl-3-oxetanylmethoxy)ethyl]phenyl ether, isobutoxymethyl(3-ethyl-3-oxetanylmethyl)ether, isobornyloxyethyl(3-ethyl-3-oxetanylmethyl)ether, isobornyl(3-ethyl-3-oxetanylmethyl)ether, 2-ethylhexyl(3-ethyl-3-oxetanylmethyl)ether, ethyldiethylene glycol (3-ethyl-3-oxetanylmethyl)ether, dicyclopentadiene(3-ethyl-3-oxetanylmethyl)ether, dicyclopentenyloxyethyl(3-ethyl-3-oxetanylmethyl)ether, dicyclopentenyl(3-ethyl-3-oxetanylmethyl)ether, tetrahydrofurfuryl(3 -ethyl-3 -oxetanylmethyl)ether, tetrabromophenyl(3 -ethyl-3 -oxetanylmethyl)ether, 2-tetrabromophenoxyethyl(3-ethyl-3-oxetanylmethyl)ether, tribromophenyl(3-ethyl-3-oxetanylmethyl)ether, 2-tribromophenoxyethyl(3-ethyl-3-oxetanylmethyl)ether, 2-hydroxyethyl(3-ethyl-3-oxetanylmethyl)ether, 2-hydroxypropyl(3-ethyl-3-oxetanylmethyl)ether, butoxyethyl(3-ethyl-3-oxetanylmethyl)ether, pentachlorophenyl(3-ethyl-3-oxetanylmethyl)ether, pentabromophenyl(3-ethyl-3-oxetanylmethyl)ether, bornyl(3-ethyl-3-oxetanylmethyl)ether, 3,7-bis(3-oxetanyl)-5-oxa-nonane, 3,3'-(1,3-(2-methylenyl)propanediyl-bis(oxymethylene))bis-(3-ethyloxetane), 1,4-bis[(3-ethyl-3-oxetanylmethoxy)methyl]benzene, 1,2-bis[(3-ethyl-3-oxetanylmethoxy)methyl]ethane, 1,3-bis[(3-ethyl-3-oxetanylmethoxy)methyl]propane, ethylene glycol-bis(3-ethyl-3-oxetanylmethyl)ether, dicyclopentenyl-bis(3-ethyl-3-oxetanylmethyl)ether, triethylene glycol-bis(3-ethyl-3-oxetanylmethyl)ether, tetraethylene glycol-bis(3-ethyl-3-oxetanylmethyl)ether, tricyclodecane-diyldimethylene(3-ethyl-3-oxetanylmethyl)ether, trimethylolpropane tris(3 -ethyl-3 -oxetanylmethyl)ether, 1,4-bis(3-ethyl-3-oxetanylmethoxy)butane, 1,6-bis(3-ethyl-3-oxetanylmethoxy)hexane, pentaerythritol tris(3-ethyl-3-oxetanylmethyl)ether, pentaerythritol tetrakis(3-ethyl-3-oxetanylmethyl)ether, polyethylene glycol-bis(3-ethyl-3-oxetanylmethyl)ether, dipentaerythritol hexakis(3-ethyl-3-oxetanylmethyl)ether, dipentaerythritol pentakis(3-ethyl-3-oxetanylmethyl)ether, dipentaerythritol tetrakis(3-ethyl-3-oxetanylmethyl)ether, caprolactone-modified dipentaerythritol hexakis(3-ethyl-3-oxetanylmethyl)ether, caprolactone-modified dipentaerythritol pentakis(3-ethyl-3-oxetanylmethyl)ether, ditrimethylolpropane tetrakis(3-ethyl-3-oxetanylmethyl)ether, EO-modified bisphenol A-bis(3-ethyl-3-oxetanylmethyl)ether, PO-modified bisphenol A-bis(3-ethyl-3-oxetanylmethyl)ether, EO-modified hydrogenated bisphenol A-bis(3-ethyl-3-oxetanylmethyl)ether, PO-modified hydrogenated bisphenol A-bis(3-ethyl-3-oxetanylmethyl)ether, and EO-modified bisphenol F-(3-ethyl-3-oxetanylmethyl)ether.

The use of these oxetane compounds is effective and thus preferred particularly when flexibility is required.

Specific examples of other compounds used as the cationically polymerizable compound include cyclic lactone compounds, such as *β*-propiolactone and *ε*-caprolactone; cyclic acetal compounds, such as trioxane, 1,3-dioxolane and 1,3,6-trioxanecyclooctane; cyclic thioether compounds, such as tetrahydrothiophene derivatives; spiro-orthoester compounds obtained by reaction between any of the above-described epoxy compounds and lactone; vinyl ether compounds, such as ethylene glycol divinyl ether, alkyl vinyl ether, 2-chloroethyl vinyl ether, 2-hydroxyethyl vinyl ether, triethylene glycol divinyl ether, 1,4-cyclohexanedimethanol divinyl ether, hydroxybutyl vinyl ether, and the propenyl ether of propylene glycol; vinyl compounds, such as ethylenically unsaturated compounds, including styrene, vinylcyclohexene, isobutylene and polybutadiene; oxolane compounds, such as tetrahydrofuran and 2,3-dimethyltetrahydrofuran; thiirane compounds, such as ethylene sulfide and thioepichlorohydrin; thietane compounds, such as 1,3-propyne sulfide and 3,3-dimethylthietane; and silicones, all of which compounds are well-known.

Further, in order to facilitate dissolution of the sulfonic acid derivative compound of the present invention into the cationically polymerizable compound, the sulfonic acid derivative compound of the present invention, prior to its use, can be dissolved in an appropriate solvent in advance (e.g., propylene carbonate, carbitol, carbitol acetate, butyrolactone or propylene glycol-1-monomethyl ether-2-acetate).

By irradiating the cationically polymerizable composition of the present invention with an energy beam such as ultraviolet radiation, the cationically polymerizable composition can be cured to a dry-to-touch state or solvent-insoluble state in usually 0.1 seconds to several minutes thereafter. As an appropriate energy beam, any energy beam may be used as long as it induces decomposition of the cationic polymerization initiator; however, it is preferred to use an electromagnetic energy beam having a wavelength of 2,000 Å to 7,000 Å that is emitted from, for example, an ultrahigh, high, medium or low-pressure mercury lamp, a xenon lamp, a carbon arc lamp, a metal halide lamp, a fluorescent lamp, a tungsten lamp, an excimer lamp, a germicidal lamp, an excimer laser, a nitrogen laser, an argon ion laser, a helium-cadmium laser, a helium neon laser, a krypton ion laser, a semiconductor laser, a YAG laser, a light-emitting diode or a CRT light source; or a high-energy beam such as an electron beam, X-ray or radiation.

The time of exposure to the energy beam is variable depending on the intensity of the energy beam, the coating film thickness and the cationically polymerizable organic compound; however, an exposure of 0.1 seconds to about 10 seconds is usually sufficient. Still, a longer irradiation time is preferred for a relatively thick coating material. By the time of 0.1 seconds to several minutes after the energy beam irradiation, the composition is mostly in a dry-to-touch state as a result of cationic polymerization; however, depending on the case, it is also preferred to use thermal energy provided by heating, a thermal head or the like in combination so as to accelerate the cationic polymerization.

Specific examples of the application of the resist composition and cationically polymerizable composition of the present invention include, but not particularly limited to: optical filters; paints; coating agents; lining agents; adhesives; printing plates; insulating varnishes; insulation sheets; laminated plates; printed circuit boards; sealants for semiconductor devices, LED packages, liquid crystal inlets, organic EL devices, optical elements, electrical insulating materials, electronic components, separation membranes and the like; molded materials; putties; glass fiber impregnants; fillers; passivation films for semiconductors, solar cells and the like; interlayer insulation films and surface protection films that are used in thin-film transistors (TFT), liquid crystal displays, organic EL displays, printed boards and the like; color filters of printed boards, color televisions, PC monitors, personal digital assistants and CCD image sensors; electrode materials for plasma display panels; printing inks; dental compositions; resins for stereolithography; liquid-form films and dry films; micromachine components; glass fiber cable coatings; materials for holographic recording; magnetic recording materials; optical switches; plating masks; etching masks; screen printing stencils; touch panels such as transparent conductive films; MEMS elements; nanoimprint materials; photofabrication applications such as two-dimensional and three-dimensional high-density mounting and the like of semiconductor packages; decoration sheets; artificial nails; glass-alternative optical films; electronic papers; optical disks; micro-lens arrays used in projectors, optical communication lasers and the like; prism lens sheets used in backlights of liquid crystal displays; Fresnel lens sheets used in the screens of projection televisions and the like; lens parts of lens sheets such as lenticular lens sheets; backlights and the like using such sheets; optical lenses such as microlenses and image pickup lenses; optical elements; optical connectors; optical waveguides; insulation packings; heat-shrinkable rubber tubes; O-rings; sealing agents for display devices; protective materials; optical fiber protection materials; adhesives; die bonding agents; high-heat radiation materials; high-heat-resistant sealing materials; members for solar cells, fuel cells and secondary batteries; solid electrolytes for batteries; insulation coating materials; heat-sensitive drums for copying machines; gas separation membranes; civil engineering and construction materials, such as concrete protecting materials, linings, soil injection agents, sealing agents, cold-heat storage materials, glass coatings and foams; medical materials such as tube/seal materials, coating materials, sealing materials for sterilizers, contact lenses, oxygen enrichment membranes and biochips; automobile components; and various mechanical components.

### EXAMPLES

The present invention will now be described in more detail by way of examples thereof; however, the present invention is not restricted by the following examples and the like by any means.

### <Synthesis Example of Sulfonic Acid Derivative Compound of Compound No. 4>

Under a nitrogen atmosphere, while stirring a solution containing 500 mL of tetrahydrofuran cooled to -70°C and 74.3 mmol of 1-butyllithium, a suspension of 750 mmol of zinc bromide and 600 ml of tetrahydrofuran was added thereto dropwise at a rate which did not increase the temperature of the reaction system above -55°C. Then, the temperature of the reaction system was restored to 15°C, and the reaction system was stirred for 1 hour to prepare a butylzinc reagent.

Under a nitrogen atmosphere, the thus obtained butylzinc reagent was added dropwise to a mixed solution of 300 mmol of 3-bromonaphthalic anhydride, 6.00 mmol of bis(diphenylphosphino)palladium dichloride (PdCl₂(dppf)₂) and 500 ml of tetrahydrofuran, and the resultant was stirred at room temperature for 1 hour. Subsequently, 1,000 mL of water was added thereto, and an organic phase was obtained by oil-water separation. To a solid phase obtained by concentrating the organic phase, 500 mL of toluene and 90.0 g of silica gel were added, and the resultant was stirred, after which a solid phase was separated by filtration. To a solid phase obtained by concentrating the resulting filtrate, 350 mL of methanol was added, and the resulting solution was heated and filtered to obtain a filtrate, which was subsequently cooled and allowed to crystallize. The resulting crystals were recovered by filtration and washed with isopropyl alcohol, after which the crystals were vacuum-dried at 45°C to obtain 26.5 g of pale-yellow crystals (3-butylnaphthalic anhydride).

The thus obtained 3-butylnaphthalic anhydride in an amount of 20.0 mmol was suspended in 30 g of dimethylformamide, and 24.0 mmol of NH₂OH-HCl was added thereto at room temperature, after which 2.00 g of 48% aqueous sodium hydroxide solution was added thereto dropwise, and the resultant was stirred for 3 hours. Further, 20.0 g of water and 0.30 g of 35% hydrochloric acid were added thereto, and the resultant was stirred for another 1 hour. The resulting precipitates were recovered by filtration, washed with a mixture of methanol and water, and then vacuum-dried at 45°C to obtain 5.06 g of hydroxyimide.

To a mixture of 10.0 mmol of the thus obtained hydroxyimide and 18.9 g of chloroform, 15.9 mmol of pyridine was added and, while maintaining the temperature of the resultant at 2°C or lower, 13.2 mmol of trifluoromethanesulfonic anhydride was further added and stirred at room temperature for 1 hour. To the resulting reaction solution, 20 g of water was added, and an oil phase obtained by oil-water separation was washed twice with 0.5% aqueous sodium hydroxide solution, once with 3% hydrochloric acid, and then five times with water. A solid phase obtained by concentrating the organic phase was heat-dissolved in chloroform, and the resultant was filtered to obtain a filtrate, which was subsequently allowed to crystallize by adding thereto methanol. The resulting crystals were recovered by filtration and vacuum-dried at 45°C to obtain 3.06 g of pale-yellow crystals. The thus obtained crystals were subjected to various analyses, and it was confirmed that the crystals were those of the sulfonic acid derivative compound of Compound No. 4. The analysis results are shown in Table 1.

**[Table 1]**

| | |
|---|---|
| Chemical shift/ppm (multiplicity, proton number) J: coupling constant (CDCl₃) | 8.61 (d,1H,J=7.3Hz), 8.54 (d,1H,J=1.8Hz), 8.26 (d,1H,J=7.9Hz), 8.09 (s,1H), 7.79 (dd,1H,J=7.9,7.3Hz), 2.92 (t,2H,J=7.6Hz), 1.80-1.70 (m,2H), 1.49-1.35 (m,2H), 0.98 (t,3H,J=7.3Hz). |
| IR absorption spectrum/cm⁻¹ | 2934, 2862, 1736, 1710, 1579, 1438, 1231, 1199, 1145, 1122, 1013, 893, 778, 718, 602 |
| Melting point (°C) | 98.2 |
| Weight reduction starting temperature (°C) | 241.0 |

### [Example 1]

### <Preparation of Positive Resist Composition>

A resin solution was prepared by dissolving 100 g of a resin having a structure in which 30 mol% of poly(p-hydroxystyrene) is substituted with *t*-butoxycarbonyl groups (Mw = 12,000) in a propylene glycol monomethyl ether acetate (PGMEA) solution, and 0.05 g of the sulfonic acid derivative compound of Compound No. 4 was dissolved in 8.00 g of this resin solution to prepare a resist solution. The thus obtained resist solution was filtered through a 0.1-µm microfilter, coated on a silicon wafer using a spin coater and then dried at 90°C for 90 seconds, after which the thus coated resist was exposed by irradiation with a light having a wavelength of 365 nm through a prescribed mask. The resultant was baked at 110°C for 90 seconds, developed by immersion in a 2.38% aqueous tetramethylammonium hydroxide solution for 30 seconds and then washed with pure water, whereby a resist pattern was obtained.

### [Example 2]

### <Preparation of Negative Resist Composition>

A resin solution was prepared by dissolving 100 g of EPPN-201 (manufactured by Nippon Kayaku Co., Ltd.) in 100 g of methyl ethyl ketone (MEK), and 0.05 g of the sulfonic acid derivative compound of Compound No. 4 was dissolved in 8.00 g of this resin solution to prepare a resist solution. The thus obtained resist solution was coated on a silicon wafer using a spin coater and then dried at 90°C for 90 seconds, after which the thus coated resist was exposed by irradiation with a light having a wavelength of 365 nm. The resultant was baked at 110°C for 90 seconds, developed by immersion in a 2.38% aqueous tetramethylammonium hydroxide solution for 30 seconds and then washed with pure water.

After the resultant was left to stand at room temperature for 24 hours, the coating film was not damaged even when it was rubbed back and forth 200 times with a cotton swab soaked in methyl ethyl ketone; therefore, it was confirmed that the curing progressed sufficiently.

### [Example 3]

### <Production of Cationically Polymerizable Composition>

To a mixture of 80 g of 3,4-epoxycyclohexylmethyl-3,4-epoxycyclohexyl carboxylate and 20 g of 1,4-butanediol diglycidyl ether, the sulfonic acid derivative compound of Compound No. 4 was added in an amount of 4 mmol, and the resultant was thoroughly stirred to homogeneity. The resulting composition was coated on a piece of aluminum-coated paper using a #3 bar coater and subsequently irradiated with the light of a 80-W/cm² high-pressure mercury lamp using a belt conveyor-equipped irradiation apparatus. The distance between the lamp and the belt conveyor was set at 10 cm, and the line speed of the belt conveyor was set at 8 m/min.

After the resultant was left to stand at room temperature for 24 hours, the coating film was not damaged even when it was rubbed back and forth 200 times with a cotton swab soaked in methyl ethyl ketone; therefore, it was confirmed that the curing progressed sufficiently.

### <Evaluation of UV Absorption Spectrum and Solubility>

The sulfonic acid derivative compound of Compound No. 4 and Comparative Compound 1 represented by the formula below were each dissolved in acetonitrile, and their absorption spectra were measured using a solar photometer U-3010 to determine the values of Xmax and ε (molar extinction coefficient) for absorption in a range of 300 nm to 400 nm as well as the value of ε at 365 nm. In addition, their solubility to propylene glycol monomethyl ether acetate at 25°C was evaluated. As for the solubility, the concentration of each compound was increased by 1%-by-mass increments, and the concentration at which no compound was left undissolved was determined to indicate the solubility in terms of this concentration. The results thereof are shown in Table 2.

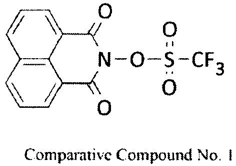

**[Table 2]**

| | Maximum absorption wavelength (nm) Molar extinction coefficient ε | Molar extinction coefficient, ε, at 365 nm | Solubility (% by mass) |
|---|---|---|---|
| Compound No. 4 | 339.5 1.31 × 10⁴ | 5.40 × 10³ | 20 |
| Comparative Compound 1 | 336.0 1.42 × 10⁴ | 0.7 × 10³ | 1.9 |

As clearly seen from Table 2, comparing the sulfonic acid derivative compound of the present invention and Comparative Compound 1, the sulfonic acid derivative compound of the present invention showed higher absorption for the light having a wavelength of 365 nm. In addition, the sulfonic acid derivative compound of Compound No. 4 had a much higher solubility than Comparative Compound 1.

### <Acid Generation Rate>

For each of the sulfonic acid derivative compound of Compound No. 4 and Comparative Compound 1, a 1.5 × 10⁻⁴ mol/L acetonitrile/water mixed solution (volume ratio: acetonitrile/water =9/1) was prepared, and exactly 5.0 mL of the mixed solution was placed in a Petri dish having an inner diameter of 42 mm. Using a UV lamp and a cut filter transmitting only light having a wavelength of about 365 nm, which are manufactured by Hoya Candeo Optronics Corporation, the mixed solution was irradiated with UV having an intensity of 100 mW/cm². The irradiation was performed for 0.5 seconds, 1 second and 3 seconds for each solution. After this exposure, the photodegradation rate (%) was determined by HPLC as the acid generation rate. The results thereof are shown in Table 3.

**[Table 3]**

| | Photodegradation rate (%) | | |
|---|---|---|---|
| | 0.5 seconds | 1 second | 3 seconds |
| Compound No. 4 | 62.0 | 86.7 | 100 |
| Comparative Compound 1 | 21.4 | 36.9 | 68.4 |

From Table 3, it was confirmed that, comparing the sulfonic acid derivative compound of Compound No. 4 whose sulfonate moiety is trifluoromethane sulfonate and Comparative Compound 1, the sulfonic acid derivative compound of the present invention (Compound No. 4) has superior acid generation capacity.

### <Evaluation of Transmittance at 365 nm>

The sulfonic acid derivative compound of Compound No. 4 and Comparative Compound 2 represented by the formula below were each dissolved in acetonitrile, and their absorption spectra were measured using a solar photometer U-3010 to determine the transmittance at 365 nm. The results thereof are shown in Table 4.

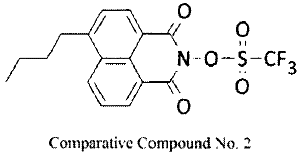

**[Table 4]**

| | Transmittance at 365 nm (%) |
|---|---|
| Compound No. 4 | 34.1 |
| Comparative Compound 2 | 1.1 |

From Table 4, it was confirmed that, comparing the sulfonic acid derivative compound of Compound No. 4 which is substituted at the 3-position of the naphthalimide skeleton and Comparative Compound 2 which is substituted at the 4-position, the sulfonic acid derivative compound of the present invention (Compound No. 4) has higher transmittance at 365 nm and expresses sufficient acid generation capacity even in the form of a thick film.

## Claims

1. A sulfonic acid derivative compound represented by the following Formula (I): 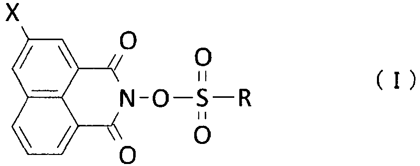 (wherein, X represents a linear or branched alkyl group having 1 to 14 carbon atoms; and R represents an aliphatic hydrocarbon group having 1 to 18 carbon atoms, an aryl group having 6 to 20 carbon atoms, an arylalkyl group having 7 to 20 carbon atoms, an acyl group-substituted aryl group having 7 to 20 carbon atoms, an alicyclic hydrocarbon group having 3 to 12 carbon atoms, a 10-camphoryl group, or a group represented by the following Formula (II), which aliphatic hydrocarbon group, aryl groups, arylalkyl group and alicyclic hydrocarbon group are optionally substituted with a group selected from the group consisting of a halogen atom, a halogenated alkyl group having 1 to 4 carbon atoms, an alkoxy group having 1 to 18 carbon atoms and an alkylthio group having 1 to 18 carbon atoms: 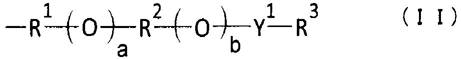 (wherein, Y¹ represents a single bond or an alkanediyl group having 1 to 4 carbon atoms; R¹ and R² each independently represent an alkanediyl group having 2 to 6 carbon atoms, a halogenated alkanediyl group having 2 to 6 carbon atoms, an arylene group having 6 to 20 carbon atoms, or a halogenated arylene group having 6 to 20 carbon atoms; R³ represents a linear or branched alkyl group having 1 to 18 carbon atoms, a linear or branched halogenated alkyl group having 1 to 18 carbon atoms, an alicyclic hydrocarbon group having 3 to 12 carbon atoms, an aryl group having 6 to 20 carbon atoms, a halogenated aryl group having 6 to 20 carbon atoms, an arylalkyl group having 7 to 20 carbon atoms, or a halogenated arylalkyl group having 7 to 20 carbon atoms; a and b each represent 0 or 1; and either a or b is 1)).

2. The sulfonic acid derivative compound according to claim 1, wherein said X is an alkyl group having 4 carbon atoms.

3. The sulfonic acid derivative compound according to claim 1 or 2, wherein said R is a perfluoroalkyl group having 1 to 8 carbon atoms.

4. A photoacid generator comprising the sulfonic acid derivative compound according to any one of claims 1 to 3.

5. A cationic polymerization initiator comprising the sulfonic acid derivative compound according to any one of claims 1 to 3.

6. A resist composition comprising the photoacid generator according to claim 4.

7. A cationically polymerizable composition comprising the cationic polymerization initiator according to claim 5.

## Patentansprüche

1. Sulfonsäurederivatverbindung, dargestellt durch die Formel (I): 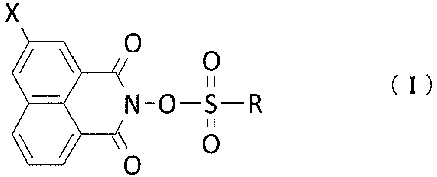 (worin X eine lineare oder verzweigte Alkylgruppe mit 1 bis 14 Kohlenstoffatomen darstellt, und R eine aliphatische Kohlenwasserstoffgruppe mit 1 bis 18 Kohlenstoffatomen, eine Arylgruppe mit 6 bis 20 Kohlenstoffatomen, eine Arylalkylgruppe mit 7 bis 20 Kohlenstoffatomen, eine Acylgruppen-substituierte Arylgruppe mit 7 bis 20 Kohlenstoffatomen, eine alicyclische Kohlenwasserstoffgruppe mit 3 bis 12 Kohlenstoffatomen, eine 10-Camphorylgruppe oder eine Gruppe, dargestellt durch die folgende Formel (II), darstellt, wobei die aliphatische Kohlenwasserstoffgruppe, Arylgruppen, Arylalkylgruppe und alicyclische Kohlenwasserstoffgruppe gegebenenfalls mit einer Gruppe substituiert sind, ausgewählt aus der Gruppe, bestehend aus einem Halogenatom, einer halogenierten Alkylgruppe mit 1 bis 4 Kohlenstoffatomen, einer Alkoxygruppe mit 1 bis 18 Kohlenstoffatomen und einer Alkylthiogruppe mit 1 bis 18 Kohlenstoffatomen: 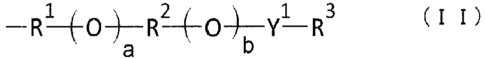 (worin Y¹ eine Einfachbindung oder eine Alkindiylgruppe mit 1 bis 4 Kohlenstoffatomen darstellt, R¹ und R² jeweils unabhängig voneinander eine Alkandiylgruppe mit 2 bis 6 Kohlenstoffatomen, eine halogenierte Alkandiylgruppe mit 2 bis 6 Kohlenstoffatomen, eine Arylengruppe mit 6 bis 20 Kohlenstoffatomen oder eine halogenierte Arylengruppe mit 6 bis 20 Kohlenstoffatomen darstellen, R³ eine lineare oder verzweigte Alkylgruppe mit 1 bis 18 Kohlenstoffatomen, eine lineare oder verzweigte halogenierte Alkylgruppe mit 1 bis 18 Kohlenstoffatomen, eine alicyclische Kohlenwasserstoffgruppe mit 3 bis 12 Kohlenstoffatomen, eine Arylgruppe mit 6 bis 20 Kohlenstoffatomen, eine halogenierte Arylgruppe mit 6 bis 20 Kohlenstoffatomen, eine Arylalkylgruppe mit 7 bis 20 Kohlenstoffatomen oder eine halogenierte Arylalkylgrupe mit 7 bis 20 Kohlenstoffatomen darstellt, a und b jeweils 0 oder 1 darstellen, und entweder a oder b 1 ist)).

2. Sulfonsäurederivatverbindung gemäß Anspruch 1, wobei das X eine Alkylgruppe mit 4 Kohlenstoffatomen ist.

3. Sulfonsäurederivatverbindung gemäß Anspruch 1 oder 2, wobei das R eine Perfluoralkylgruppe mit 1 bis 8 Kohlenstoffatomen ist.

4. Photosäuregenerator, umfassend die Sulfonsäurederivatverbindung gemäß einem der Ansprüche 1 bis 3.

5. Kationischer Polymerisationsinitiator, umfassend die Sulfonsäurederivatverbindung gemäß einem der Ansprüche 1 bis 3.

6. Resistzusammensetzung, umfassend den Photosäuregenerator gemäß Anspruch 4.

7. Kationische polymerisierbare Zusammensetzung, umfassend den kationischen Polymerisationsinitiator gemäß Anspruch 5.

## Revendications

1. Composé de type dérivé d'acide sulfonique représenté par la formule (I) suivante : 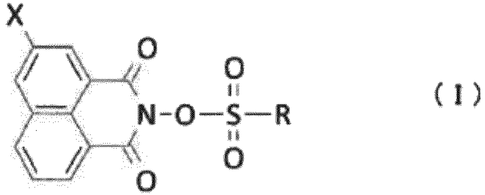 où X représente un groupe alkyle linéaire ou ramifié ayant 1 à 14 atomes de carbone ; et R représente un groupe hydrocarboné aliphatique ayant 1 à 18 atomes de carbone, un groupe aryle ayant 6 à 20 atomes de carbone, un groupe arylalkyle ayant 7 à 20 atomes de carbone, un groupe aryle substitué par un groupe acyle ayant 7 à 20 atomes de carbone, un groupe hydrocarboné alicyclique ayant 3 à 12 atomes de carbone, un groupe 10-camphoryle, un groupe représenté par la formule (II) suivante, où le groupe hydrocarboné aliphatique, les groupes aryle, le groupe arylalkyle et le groupe hydrocarboné alicyclique sont le cas échéant substitués par un groupe choisi parmi le groupe consistant en un atome d'halogène, un groupe alkyle halogéné ayant 1 à 4 atomes de carbone, un groupe alcoxy ayant 1 à 18 atomes de carbone et un groupe alkylthio ayant 1 à 18 atomes de carbone : 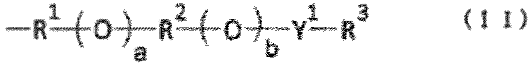 où Y¹ représente une simple liaison ou un groupe alcanediyle ayant 1 à 4 atomes de carbone ; R¹ et R² représentent chacun indépendamment, un groupe alcanediyle ayant 2 à 6 atomes de carbone, un groupe alcanediyle halogéné ayant 2 à 6 atomes de carbone, un groupe arylène ayant 6 à 20 atomes de carbone, ou un groupe arylène halogéné ayant 6 à 20 atomes de carbone ; R³ représente un groupe alkyle linéaire ou ramifié ayant 1 à 18 atomes de carbone, un groupe alkyle halogéné linéaire ou ramifié ayant 1 à 18 atomes de carbone, un groupe hydrocarboné alicyclique ayant 3 à 12 atomes de carbone, un groupe aryle ayant 6 à 20 atomes de carbone, un groupe aryle halogéné ayant 6 à 20 atomes de carbone, un groupe arylalkyle ayant 7 à 20 atomes de carbone, ou un groupe arylalkyle halogéné ayant 7 à 20 atomes de carbone ; a et b représentent chacun, 0 ou 1, et a ou b est 1.

2. Composé de type dérivé d'acide sulfonique selon la revendication 1, où ledit X est un groupe alkyle ayant 4 atomes de carbone.

3. Composé de type dérivé d'acide sulfonique selon la revendication 1 ou 2, où ledit R est un groupe perfluoroalkyle ayant 1 à 8 atomes de carbone.

4. Photogénérateur d'acide comprenant le composé de type dérivé d'acide sulfonique selon l'une quelconque des revendications 1 à 3.

5. Initiateur de polymérisation cationique comprenant le composé de type dérivé d'acide sulfonique selon l'une quelconque des revendications 1 à 3.

6. Composition de réserve comprenant le photogénérateur d'acide selon la revendication 4.

7. Composition polymérisable de manière cationique, comprenant l'initiateur de polymérisation cationique selon la revendication 5.
